(19) Europäisches Patentamt
European Patent Office
Office européen des brevets

(11) Numéro de publication: **0 389 369 B1**

(12) # FASCICULE DE BREVET EUROPEEN

(45) Date de publication de fascicule du brevet: **09.11.94**

(51) Int. Cl.[5]: **C07J 43/00**, C07J 1/00, A61K 31/565

(21) Numéro de dépôt: **90400783.8**

(22) Date de dépôt: **22.03.90**

(54) **Nouveaux stéroides 3-céto comportant une chaîne en 17 aminosubstituée, leur procédé de préparation et les intermédiaires de ce procédé, leur application comme médicaments et les compositions les contenant.**

(30) Priorité: **22.03.89 FR 8903741**

(43) Date de publication de la demande:
**26.09.90 Bulletin 90/39**

(45) Mention de la délivrance du brevet:
**09.11.94 Bulletin 94/45**

(84) Etats contractants désignés:
**CH DE FR GB IT LI NL**

(56) Documents cités:
**WO-A-87/01706**
**WO-A-87/07895**
**GB-A- 1 169 073**
**GB-A- 2 025 422**

(73) Titulaire: **ROUSSEL-UCLAF**
**35, Boulevard des Invalides**
**F-75007 Paris (FR)**

(72) Inventeur: **Claussner, André**
**62, rue Marc Vieville**
**F-93250 Villemomble (FR)**
Inventeur: **Leclaire, Jacques**
**1, rue de l'Epine Montain**
**F-91300 Massy (FR)**
Inventeur: **Nedelec, Lucien**
**45, Boulevard de l'Ouest**
**F-93340 Le Raincy (FR)**
Inventeur: **Philibert, Daniel**
**16, rue Chevalier**
**F-94210 La Varenne Saint Hilaire (FR)**

(74) Mandataire: **Bourgouin, André et al**
**Département des Brevets**
**ROUSSEL UCLAF**
**111, route de Noisy**
**B.P. no 9**
**F-93230 Romainville (FR)**

**EP 0 389 369 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouveaux stéroïdes 3-céto comportant une chaîne en 17 aminosubstituée, leur procédé de préparation et les intermédiaires de ce procédé, leur application comme médicaments et les compositions pharmaceutiques les contenant.

L'invention a pour objet, les composés de formule (I) :

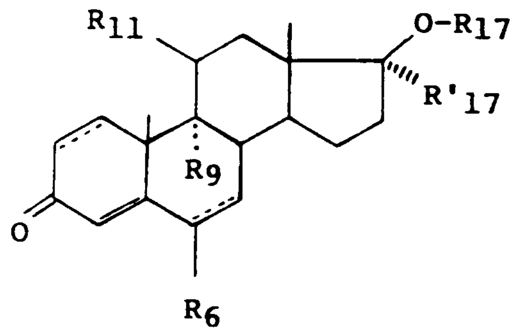

(I)

dans laquelle :

- $R_6$ représente un atome d'hydrogène, un radical méthyle, un atome de fluor ou un atome de chlore,
- $R_9$ et $R_{11}$ forment ensemble une seconde liaison entre les carbones $C_9$ et $C_{11}$ ou $R_9$ est un atome d'hydrogène ou un atome de fluor et $R_{11}$ est un atome d'hydrogène, un radical hydroxyle ou un radical oxo,
- les traits pointillés, dans les cycles A et B, indiquent la présence éventuelle d'une liaison supplémentaire en position 1(2) et 6(7)

et dans laquelle $R_{17}$ est un atome d'hydrogène ou un radical acyle dérivé d'un acide organique carboxylique renfermant de 1 à 18 atomes de carbone et $R'_{17}$ est un radical

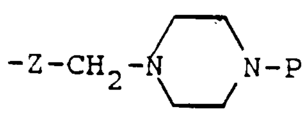

dans lequel Z est une simple liaison, un radical alcénylène ou un radical alcynylène renfermant de 2 à 5 atomes de carbone et P représente un radical pyrimidinyle ou un radical pyridyle, éventuellement substitué par un ou deux radicaux identiques ou différents choisis parmi les radicaux amino, alkyl ou dialkylamino ou les hétérocycles aminés à 5 ou 6 chaînons, substitués par un radical alkyle ayant de 1 à 3 atomes de carbone, et les sels de ces composés.

Lorsque $R_{17}$ est un radical acyle, il peut s'agir du dérivé d'un acide aliphatique ou cycloaliphatique saturé ou insaturé et notamment d'un acide alcanoïque tel que par exemple l'acide acétique, propionique, butyrique ou isobutyrique, valérique ou undécylique, d'un acide hydroxyalcanoïque tel que par exemple l'acide hydroxyacétique ; d'un acide cycloalkylcarboxylique ou (cycloalkyl) alcanoïque tel que par exemple l'acide cyclopropyl, cyclopentyl ou cyclohexylcarboxylique, cyclopentyl ou cyclohexyl acétique ou propionique, d'un acide benzoïque ou d'un acide phénylalcanoïque tel que l'acide phényl acétique ou phényl propionique, d'un amino acide tel que l'acide diéthylamino acétique ou aspartique ou de l'acide formique ; il s'agit de préférence du dérivé de l'acide acétique.

Dans $R'_{17}$, lorsque Z est un radical alcénylène il s'agit de préférence d'un radical vinylène, lorsque Z est un radical alcynylène il s'agit de préférence d'un radical éthynylène et lorsque P est substitué par un radical alkylamino, le radical alkyle comporte de préférence, de 1 à 4 atomes de carbone, il peut s'agir des radicaux méthyl, éthyl, n-propyl, isopropyl, n-butyl, isobutyl, tert-butylamino, de préférence du radical méthyl ou éthylamino ; lorsque P est substitué par un radical dialkylamino, les radicaux alkyles comportent de préférence, de 1 à 4 atomes de carbone, il peut s'agir des radicaux diméthyl, diéthyl ou méthyléthylamino ; lorsque P est substitué par un ou deux hétérocycles aminés il peut s'agir d'un hétérocycle saturé, de préférence une pyrrolidine ou une pipéridine, éventuellement substitué par un radical alkyle tel que méthyle, éthyle, propyle ou isopropyle, de préférence méthyle ou éthyle, ou d'un hétérocycle insaturé, de préférence un pyrrole éventuellement substitué par un radical alkyle tel qu'un radical méthyle.

L'invention s'étend naturellement aux sels des composés de formule (I), comme par exemple les sels formés avec les acides chlorhydrique, bromhydrique, nitrique, sulfurique, phosphorique, acétique, formique, propionique maléïque, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques, tels que l'acide benzoïque.

Parmi les composés de l'invention, on peut citer particulièrement les composés de formule (I) pour lesquels P représente un radical pyrimidinyle ou un radical pyridyle substitué par deux radicaux identiques ou différents choisis parmi les radicaux dialkylamino ou les hétérocycles aminés à 5 ou 6 chaînons et tout particulièrement les composés de formule (I) pour lesquels P représente un radical 2,6-bis(1-pyrrolidinyl) 4-pyrimidinyle, un radical 5,6-bis(diéthylamino) 2-pyridyle ou un radical 3,6-bis(diéthylamino) 2-pyridyle.

Parmi les composés de l'invention, on peut citer notamment les composés de formule (I) pour lesquels $R_6$ est un atome d'hydrogène.

L'invention a tout spécialement pour objet les composés de formule (I) pour lesquels $R_6$ est un radical méthyle ainsi que ceux pour lesquels le trait en pointillé en position 6(7) indique la présence d'une seconde liaison.

Parmi les composés préférés de l'invention on peut citer ceux pour lesquels $R_9$ est un atome d'hydrogène et $R_{11}$ est un radical hydroxyle, ceux pour lesquels le trait en pointillé en position 1(2) indique la présence d'une seconde liaison et plus particulièrement ceux pour lesquels $R_{17}$ est un atome d'hydrogène et ceux pour lesquels $R'_{17}$ représente un radical

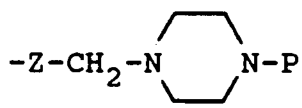

dans lequel Z représente un radical -C≡C-.

Parmi les composés préférés de l'invention, on peut donc naturellement citer les composés dont la préparation est donnée plus loin dans la partie expérimentale à savoir :

- la 17alpha-[3-[4-[2,6-bis(1-pyrrolidinyl) 4-pyrimidinyl] 1-pipérazinyl] 1-propynyl] 11béta,17béta-dihydroxy-androsta 4,6-diène-3-one, ou
- la 17alpha-[3-[4-[2,6-bis(1-pyrrolidinyl) 4-pyrimidinyl] 1-pipérazinyl] 1-propynyl] 11béta,17béta-dihydroxy 6-méthyl-androsta-1,4,6-trièn-3-one ou
- la 17alpha-[3-[4-[5,6-bis(diéthylamino) 2-pyridyl] 1-pipérazinyl] 1-propynyl] 11béta,17beta-dihydroxy 6-méthyl androsta-1,4,6-trièn-3-one ou
- la 17alpha-[3-[4-[3,6-bis(diéthylamino) 2-pyridyl] 1-pipérazinyl] 1-propynyl] 11béta,17béta-dihydroxy 6-méthyl androsta-1,4,6-trièn-3-one

et leurs sels.

L'invention a également pour objet un procédé de préparation des composés de formule (I) caractérisé en ce que l'on soumet, dans un solvant neutre et en présence d'une base, un composé de formule (II) :

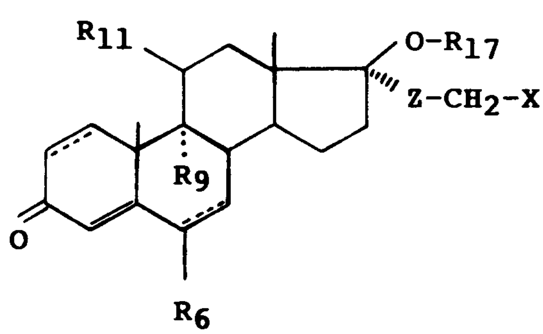

(II)

dans laquelle X est un atome d'halogène et $R_6$, $R_9$, $R_{11}$, les traits pointillés, $R_7$ et Z ont la signification indiquée précédemment, à l'action d'un composé de formule (III) :

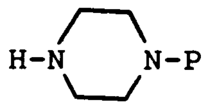

(III)

dans laquelle P a la même signification que précédemment et, si désiré, l'on soumet les produits obtenus à l'action d'un acide pour obtenir le sel correspondant.

Les composés de formule (I) sont obtenus en utilisant un composé de formule (II) dans lequel X, peut être un atome de chlore, de brome ou d'iode et en opérant dans un solvant neutre tel que par exemple le diméthylformamide, le tétrahydrofuranne, le chlorure de méthylène, l'acétonitrile, l'éther éthylique ou l'acétone, en présence d'une base telle que par exemple un carbonate ou un bicarbonate de métal alcalin de préférence de sodium ou de potassium, la triéthylamine ou la diisopropyléthylamine.

Dans un mode de réalisation préféré du procédé de l'invention, le composé de formule (II) est soit la :

- 17alpha-(3-bromo-1-propynyl) 11béta,17béta-dihydroxy androsta-4,6-dièn-3-one, soit la :
- 17alpha-(3-bromo-1-propynyl) 11béta,17béta-dihydroxy 6-méthylandrosta-1,4,6-trièn-3-one, le solvant neutre est l'acétone, la base est le carbonate de potassium et le composé de formule (III) est soit la :
- 2,4-bis(1-pyrrolidinyl) 6-(1-pipérazinyl) pyrimidine

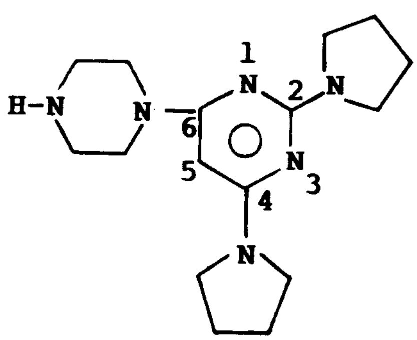

qui peut être obtenue selon la préparation décrite dans la demande de brevet WO 87/01706, soit la :

- N,N,N′,N′-tétraéthyl 6-(1-pipérazinyl) 2,3-pyridinediamine

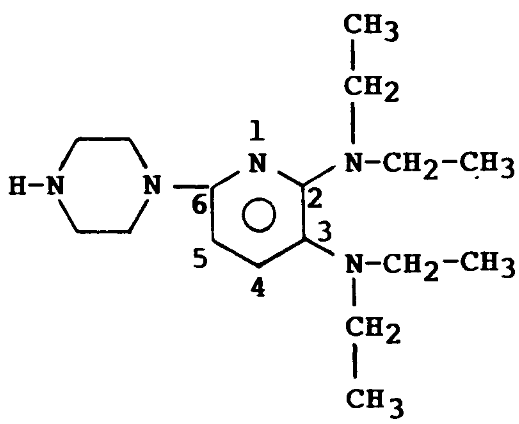

qui, bien que dénommée de façon erronée, peut être obtenue selon la préparation décrite dans la demande de brevet WO 87/01706

ou soit la :

- N,N,N′,N′-tétraéthyl 6-(1-pipérazinyl) 2,5-pyridinediamine

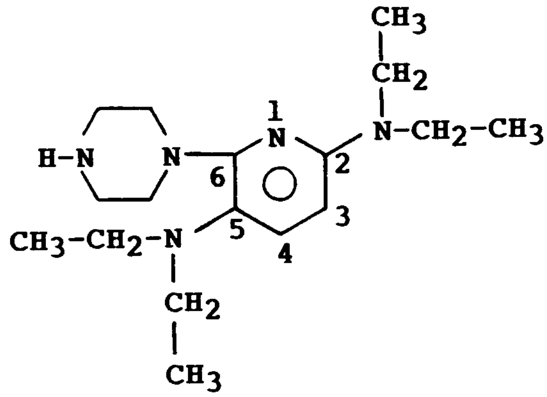

décrite dans la demande de brevet français n° 89-03740 et dont la préparation est donnée plus loin dans la partie expérimentale et qui, contrairement à ce qui est indiqué, ne peut être obtenue selon la préparation décrite dans la demande de brevet WO 87/01706.

Les composés de formule (I) selon l'invention peuvent être obtenus à l'état de sels par des méthodes connues qui consistent à faire réagir le composé (I) avec un acide minéral tel que par exemple l'acide chlorhydrique, bromhydrique, nitrique, sulfurique, phosphorique, ou avec un acide organique tel que par exemple l'acide acétique, formique, propionique, maléïque, fumarique, succinique, tartrique, citrique, oxalique, glyoxylique, aspartique ou bien un acide alcane sulfonique tel que l'acide méthane ou éthane sulfonique, arylsulfonique, tel que l'acide benzène ou paratoluène sulfonique et arylcarboxylique, tel que l'acide benzoïque.

Les acides préférés, selon l'invention, sont l'acide méthanesulfonique ou l'acide fumarique.

Les composés de formule (I) ainsi que leurs sels présentent d'intéressantes propriétés pharmacologiques, notamment, une activité antioxydante par inhibition de la péroxydation lipidique tissulaire, par exemple au niveau du rein, du coeur, plus particulièrement au niveau du cerveau et de la moelle épinière. Les composés de formule (I) ainsi que leurs sels présentent aussi une activité détoxifiante dans les intoxications aiguës associées à la péroxydation des lipides des tissus cérébraux tels que le cerveau ou la moelle épinière.

Les composés de formule (I) ainsi que leurs sels présentent en outre une activité anti-inflammatoire intéressante, par exemple dans les phénomènes de l'inflammation aiguë médiée par les dérivés de l'acide arachidonique.

Ces propriétés justifient leur application en thérapeutique.

L'invention a donc pour objet les composés de formule (I), à titre de médicaments, ainsi que leurs sels avec les acides pharmaceutiquement acceptables.

Parmi les médicaments de l'invention, on peut citer tout particulièrement les composés des exemples 1, 2, 3 et 4 et leurs sels avec les acides pharmaceutiquement acceptables.

Les médicaments selon l'invention, peuvent être utilisés dans le traitement de désordres biologiques consécutifs à des traumatismes. On entend, par traumatismes, des dommages tissulaires dans lesquels interviennent la génération de péroxydes lipidiques et qui peuvent être provoqués par des agents variés, par exemple des agents physiques tels que des contusions, en particulier des contusions cérébrales associées ou non à des hémorragies locales, ou des agents chimiques tels que ceux utilisés en chimiothérapie antitumorale comme l'adriamycine ou tels que ceux utilisés en immunothérapie cancéreuse comme l'IL2 ou le TNF.

Ils sont surtout intéressants dans le traitement de l'ischémie cérébrale, en particulier dans le traitement de l'infarctus cérébral et dans la prévention de sa récidive, ou dans le traitement d'intoxications médicamenteuses provoquées par une chimiothérapie ou une immunothérapie ou l'association des deux.

Ils peuvent en outre être utilisés dans le traitement des réactions inflammatoires ; ils sont intéressants pour le traitement des réactions inflammatoires locales, par exemple les oedèmes, les dermatoses, les prurits, les diverses formes d'eczéma et les érythèmes solaires ou pour le traitement de maladies inflammatoires aiguës ou les maladies inflammatoires chroniques, par exemple la polyarthrite rhumatoïde, le psoriasis ou la sclérose en plaque.

Les médicaments de l'invention peuvent être administrés par voie orale ou par voie parentérale, telle qu'en injection intra-musculaire, intraarticulaire, intrathécale, de préférence en injection intraveineuse en

bolus ou en perfusion continue ou par voie locale en application topique sur la peau ou les muqueuses.

L'invention s'étend aux compositions pharmaceutiques renfermant comme principe actif au moins l'un quelconque des médicaments ci-dessus.

Les compositions pharmaceutiques sont préparées selon les méthodes usuelles. Le principe actif peut être incorporé à des excipients habituellement employés dans ces compositions pharmaceutiques, tels que le talc, l'amidon, les véhicules aqueux ou non, les corps gras d'origine animale ou végétale, les dérivés paraffiniques, les glycols, les divers agents mouillants, dispersants ou émulsifiants et les conservateurs.

Les sels des produits de formule (I), plus solubles dans l'eau, sont utilisés de préférence pour les formulations aqueuses destinées à l'injection intraveineuse.

La posologie utile, la fréquence et la durée du traitement qui dépendent de l'aminostéroïde et de la voie d'administration choisis varient, notamment en fonction du sujet et de la sévérité de l'affection à traiter. La dose peut être comprise entre 0,02 et 100 mg/kg/j, de préférence entre 0,01 et 1 mg/kg/j par voie intraveineuse ou par voie intra-musculaire et éventuellement répétée plusieurs fois par jour.

Les composés de formule (II) utilisés comme produits de départ sont des dérivés halogénés de stéroïdes 3-céto qui sont des produits nouveaux et l'invention a donc également pour objet les composés de formule (II) à titre de produits industriels nouveaux.

Parmi les produits nouveaux de formule (II) préférés de l'invention, on peut citer les composés dont la préparation est donnée plus loin dans la partie expérimentale, à savoir :

- la 17alpha-(3-bromo-1-propynyl) 11béta,17béta-dihydroxy-androsta-4,6-dièn-3-one
  ou :
- la 17alpha-(3-bromo-1-propynyl) 11béta,17béta-dihydroxy 6-méthylandrosta-1,4,6-trièn-3-one.

Les composés de formule (II) sont préparés selon un mode opératoire dont deux exemples sont donnés plus loin dans la partie expérimentale.

De manière générale, les composés de formule (II) peuvent être préparés selon le schéma suivant :

Selon le procédé décrit dans la demande de brevet FR 2 433 536, on fait agir le lithien dérivé du produit de formule (IV) :

$H-Z-CH_2-OR$ (IV)

dans laquelle Z a la signification donnée ci-dessus et R représente un groupement protecteur de la fonction alcool, sur un stéroïde de formule (V) :

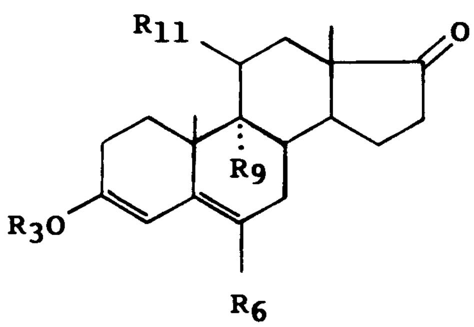

(V)

dans laquelle $R_6$, $R_9$ et $R_{11}$ ont la signification indiquée ci-dessus et dans laquelle $R_3$ représente un radical alkyle ayant de 1 à 4 atomes de carbone pour obtenir un produit de formule (VI) :

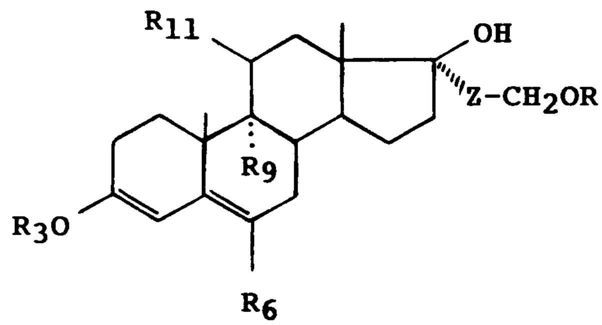

(VI)

que :

- **soit** l'on soumet à un agent capable de libérer la fonction cétone et de créer le système de liaison delta-4,6, par exemple le chloranile ou la 2,3-dichloro-5,6-dicyanobenzoquinone (DDQ), pour obtenir un produit de formule (VII) :

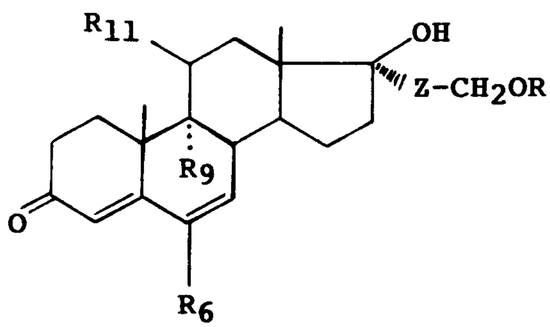

(VII)

- **soit** l'on débloque la fonction cétone en 3 par hydrolyse acide puis, si désiré, on soumet le produit 3-céto delta-4 obtenu à un agent de formation de la double liaison en 1(2) tel qu'un micro-organisme, par exemple Arthrobacter Simplex ou bien une quantité stoechiométrique de DDQ puis si désiré à une deuxième quantité stoechiométrique de DDQ pour obtenir une liaison supplémentaire en 6(7), puis on débloque la fonction alcool par hydrolyse acide pour obtenir le produit de formule (VIII) :

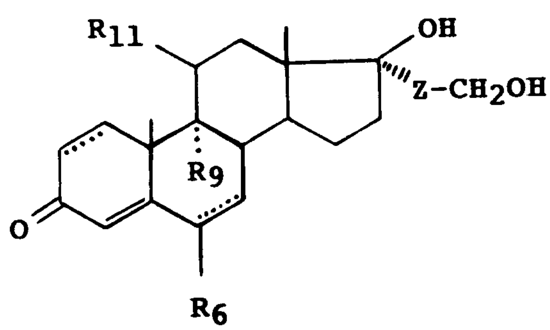

(VIII)

que si désiré, lorsque Z est un radical alcynylène ou acénylène, l'on soumet à un agent d'hydrogénation partielle ou totale, exemple à l'aide d'hydrogène en présence de palladium sur charbon actif ou sur sulfate de baryum en présence ou non de triéthylamine afin d'obtenir les produits correspondants ayant un radical Z alcénylène ou alkylène et que l'on soumet à un agent d'halogénation tel qu'un halogénure comme le tétrabromure de carbone pour obtenir un produit de formule correspondant à un produit de formule (II) dans laquelle $R_{17}$ est un atome d'hydrogène. Les produits obtenus peuvent être ensuite soumis, si désiré, à un agent d'estérification, par exemple selon le procédé décrit dans la demande de brevet FR 2 433 536, qui introduit le radical acyle en position 17 seulement.

Les produits de formule (V) nécessaires à la mise en oeuvre du procédé sont des stéroïdes 17-céto qui sont des produits connus décrits par exemple dans les brevets USP 2.775.602, 2.793.218, 4.189.431, 3.505.365 ou 2.656.370.

Les stéroïdes de formule (V) ayant une double liaison en 9(11) sont des produits connus ou préparés selon les méthodes connues de l'homme de métier, par exemple par deshydratation d'un stéroïde 11-hydroxylé correspondant par un mélange de chlorure de méthanesulfonyle et de chlorure de thionyle.

Les stéroïdes de formule (V) ayant un radical méthyle en $C_6$ sont préparés selon des méthodes connues de l'homme de métier, par exemple par action d'un halogénure de méthyl-magnésium sur un 5(6)-époxy stéroïde correspondant ayant une cétone en position 3 bloquée par exemple par un groupement cétal.

Les stéroïdes de formule (V) ayant un atome de fluor en $C_6$ sont préparés selon des méthodes connues, par exemple par addition du complexe acide fluorhydrique-diméthylformamide sur un 5(6)-époxystéroïde correspondant ayant une cétone en position 3 bloquée par exemple par un groupement cétal.

Les stéroïdes de formule (V) ayant un atome de chlore en $C_6$ sont préparés selon des méthodes connues, par exemple par addition d'acide chlorhydrique gazeux dissous dans l'acide acétique sur un 5(6)-époxystéroïde correspondant ayant une cétone en position 3 bloquée par un groupement cétal.

Les stéroïdes de formule (V) ayant un atome de fluor en $C_9$ sont des produits connus ou préparés selon des méthodes connues, par exemple par addition du complexe acide fluorhydrique-diméthylformamide sur un 9(11)-époxystéroïde correspondant ayant une cétone en position 3, éventuellement bloquée par exemple par un groupement cétal.

Les composés de formule (III) utilisés comme produits de départ du procédé de l'invention, dans lesquels P est un radical non substitué ou monosubstitué, sont préparés selon la demande de brevet WO 87/1706 ainsi que les composés de formule (III) dans lesquels P est un radical disubstitué tel qu'un radical 2,6-(disubstitué) 4-pyrimidinyle, par exemple le radical 2,6-bis(1-pyrrolidinyl) 4-pyrimidinyle :

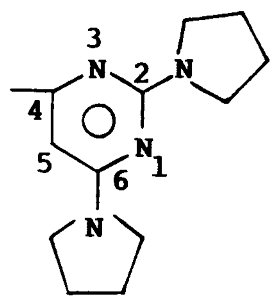

ou un radical 5,6-(disubstitué) 2-pyridyle, par exemple le radical 5,6-bis(diéthylamino) 2-pyridyle :

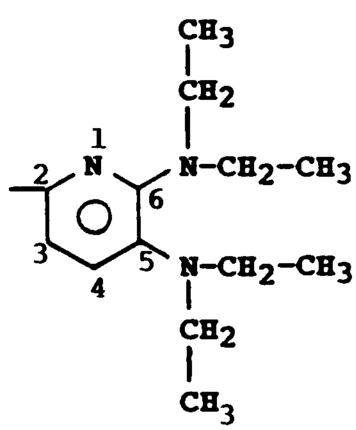

Par contre, les composés de formule (III) dans lesquels P est un radical 3,6-(disubstitué) 2-pyridyle, par exemple le radical 3,6-bis(diéthylamino) 2-pyridyle :

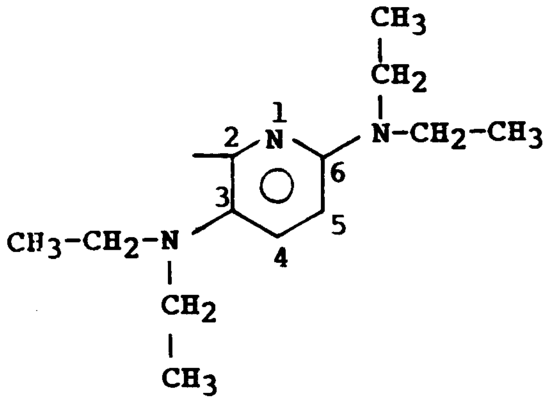

sont des produits nouveaux qui sont préparés tel que décrit dans la demande de brevet français n° 89-03740, selon un mode opératoire dont un exemple de préparation est donné plus loin dans la partie expérimentale.

Ces composés de formule (III) peuvent être préparés selon le schéma suivant :
On fait agir un produit de formule (IX) :

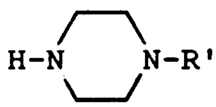

(IX)

dans laquelle R′ représente un groupement protecteur de la fonction amino, par exemple un radical acétyle, sur un produit de formule (X) :

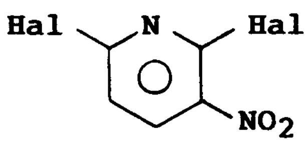

(X)

dans laquelle Hal représente un atome d'halogène, de préférence un atome de chlore, pour obtenir un produit de formule (XI) :

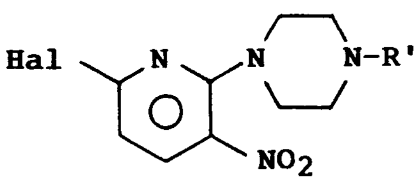

(XI)

sur lequel on fait réagir un produit de formule (XII) :

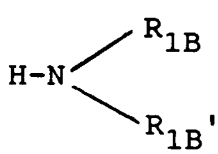

(XII)

dans laquelle $R_{1B}$ et $R_{1B}'$ ont soit la signification indiquée ci-dessus pour $R_B$ et $R_B'$ soit sont tels que, ou bien l'un représente un groupement protecteur monovalent de la fonction amino, par exemple un groupement benzyle ou un groupement trityle, et l'autre représente un atome d'hydrogène ou bien $R_{1B}$ et $R_{1B}'$ représentent ensemble un groupement protecteur divalent, par exemple $R_{1B}$ et $R_{1B}'$ forment ensemble avec

l'azote auquel ils sont liés un 2,5-diméthylpyrrole, pour obtenir un produit de formule (XIII) :

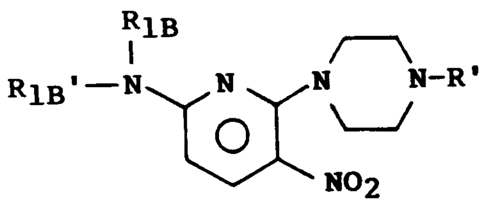

(XIII)

que l'on soumet à une réaction d'hydrogénation pour obtenir un produit de formule (XIV) :

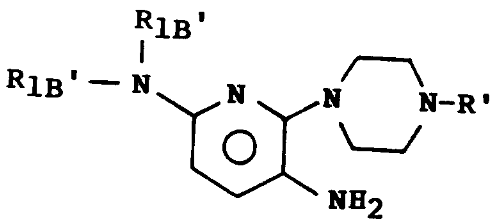

(XIV)

produit que si désiré :

- **soit a)** l'on soumet à l'action d'un ou deux équivalents d'un derivé monohalogéné de $R_A$ ou de $R_A'$pour obtenir un produit de formule (XIV') :

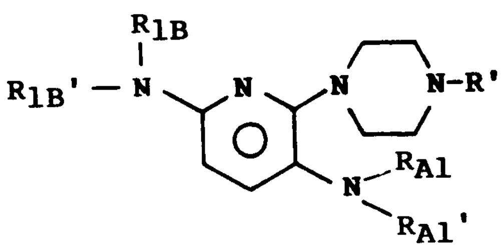

(XIV')

dans laquelle $R_{A1}$ et $R_{A1}'$ représentent soit l'un un atome d'hydrogène et l'autre un radical alkyle, soit tous les deux le même radical alkyle,

- **soit b)** l'on soumet à l'action d'un dérivé monohalogéné de $R_A$ ou de $R_A'$, puis à l'action d'un dérivé monohalogéné de $R_A'$ ou de $R_A$ pour obtenir un produit de formule (XIV") :

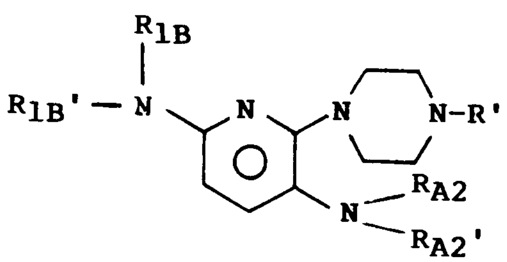

(XIV'')

dans laquelle $R_{A2}$ et $R_{A2}'$ représentent des radicaux alkyles différents,

- **soit c)** l'on soumet à l'action d'un dérivé dihalogéné du butane ou du pentane, éventuellement substitué par un radical alkyle ayant de 1 à 3 atomes de carbone pour obtenir un produit de formule (XIV‴) :

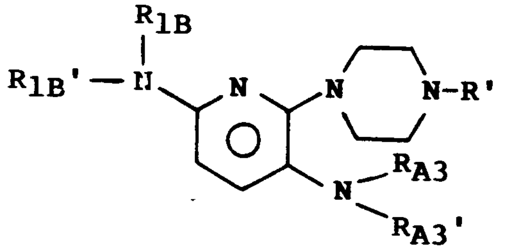

(XIV''')

dans laquelle $R_{A3}$ et $R_{A3}'$ forment avec l'atome auquel ils sont liés un hétérocycle à 5 ou 6 chaînons, produits de formule (XIV), (XIV'), (XIV'') et (XIV''') que l'on soumet à une réaction de déblocage du groupement R' et éventuellement des groupements $R_{1B}$ et/ou $R_{1B}'$ pour obtenir les composés de formule (III) correspondants.

Parmi les produits nouveaux de formule (III) préférés de l'invention, on peut citer le composé dont la préparation est donnée plus loin dans la partie expérimentale, à savoir la N,N,N',N'-tétraéthyl 6-(1-pipérazinyl) 2,5-pyridinediamine qui, comme indiqué précédemment, ne peut être obtenue selon la préparation décrite dans la demande de brevet WO 87/01706. On peut egalement citer le composé de formule (III) à savoir la N,,N'-dipyrrolidyl 6-(1-pipérazinyl) 2,5-pyridinediamine.

Les exemples suivants illustrent l'invention sans toutefois la limiter.

**Préparation 1 : 17alpha-(3-bromo 1-propynyl) 11béta,17béta-dihydroxy androsta-4,6-dièn-3-one.**

Stade A : 3-éthoxy 11béta,17béta-dihydroxy 17alpha-(3-tétrahydropyrannyloxy 1-propynyl) androsta-3,5-diène.

A 17,2 $cm^3$ d'une solution 1,6 M de méthyl lithium dans l'éther, refroidie à -5°/-7°C, on ajoute sous agitation, une solution de 4,27 g de tétrahydro 2-(2-propynyloxy) 2H-pyranne dans 13 $cm^3$ d'éther éthylique. Après 30 minutes d'agitation à -5°C/-7°C, on ajoute en 1 heure, une solution de 2,03 g de 3-éthoxy 11béta-hydroxy androsta-3,5-dièn-17-one, (préparé selon le brevet japonais 9577/71) dans 14 $cm^3$ de tétrahydrofuranne. On laisse revenir à température ambiante et agite 4 heures. On refroidit et ajoute 20 $cm^3$ d'une solution aqueuse saturée de chlorure d'ammonium. On extrait avec de l'acétate d'éthyle et concentre à sec sous pression réduite. On obtient 5,5 g de produit brut que l'on chromatographie sur silice (éluant : cyclohexane-acétate d'éthyle (6-4)). On obtient 1,57 g de produit attendu.

Spectre IR ($CHCl_3$)

- OH 3604 $cm^{-1}$

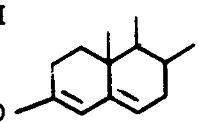

- RO 1653 $cm^{-1}$

1627 $cm^{-1}$

Stade B : 11béta,17béta-dihydroxy 17alpha-(3-tétra-hydropyrannyloxy 1-propynyl) androsta-4,6-dièn-3-one.

A une solution de 8,2 g de produit obtenu au stade A dans 164 $cm_3$ d'acétone à 5 % d'eau, on ajoute 8,2 g de chloranile et on agite 4 heures à température ambiante. On coule le mélange réactionnel dans 400 $cm^3$ d'un mélange à parties égales de bicarbonate de potassium en solution aqueuse saturée et de thiosulfate de sodium à 10 % dans l'eau. On extrait avec du chlorure de méthylène et concentre à sec sous pression réduite, on obtient 8,6 g de produit brut que l'on chromatographie sur silice (éluant : cyclohexane-acétate d'éthyle (4-6)). On recueille 6,65 g de produit recherché.

| Spectre IR ($CHCl_3$) | |
|---|---|
| Delta-4,6 3-one | 1654 $cm^{-1}$<br>1618 $cm^{-1}$<br>1584 $cm^{-1}$<br>878 $cm^{-1}$ |
| OH bande forte<br>C-O-C | 3611 $cm^{-1}$ |

Stade C : 11béta,17béta-dihydroxy 17alpha-(3-hydroxy 1-propynyl) androsta-4,6-dièn-3-one.

A une solution de 8,9 g de produit obtenu comme au stade B dans 178 $cm^3$ d'éthanol, on ajoute 44 $cm^3$ d'acide chlorhydrique 2N, on agite pendant 1 heure et 30 minutes à température ambiante. On refroidit et ajoute 10 $cm^3$ d'ammoniaque concentré. On distille l'éthanol sous pression réduite, extrait à l'acétate d'éthyle et amène à sec sous pression réduite. Le résidu (7,89 g) est chromatographié sur silice (éluant : acétate d'éthyle-cyclohexane (9-1)). On obtient 6,16 g de produit brut que l'on purifie par empâtage dans 10 vol d'éther isopropylique, on recueille 5,94 g de produit recherché. F = 218 °C.

| Analyse pour $C_{22}H_{28}O_4$ : | | | | |
|---|---|---|---|---|
| Calculés<br>Trouvés | C% | 74,12<br>73,8 | H% | 7,91<br>8,0 |

| Spectre IR ($CHCl_3$) | |
|---|---|
| Delta-4,6 3-one | 1655 $cm^{-1}$ (F)<br>1608 $cm^{-1}$<br>1584 $cm^{-1}$<br>878 $cm^{-1}$ |
| OH | 3611 $cm^{-1}$ (F) |

Stade D : 17alpha-(3-bromo 1-propynyl) 11béta,17béta-dihydroxy androsta-4,6-dièn-3-one.

A une solution de 5 g de produit obtenu au stade C dans 75 $cm^3$ de chlorure de méthylène, on ajoute 6,96 g de tétrabromure de carbone. A -5 °C on ajoute lentement une solution de 5,5 g de triphényl phosphine dans 27 $cm^3$ de chlorure de méthylène. On agite 30 minutes en laissant la température revenir à +5 °C, on chromatographie le milieu réactionnel sur silice (éluant : cyclohexane-acétate d'éthyle (1-1)). On obtient 9,92 g de produit que l'on chromatographie à nouveau sur silice (éluant : chlorure de méthylène-méthanol (95-5)). On obtient 5,43 g de produit recherché. F = 210 °C après recristallisation d'un mélange chlorure de méthylène-éther isopropylique.

| Analyse pour $C_{22}H_{27}O_3Br$ : | | | | | | |
|---|---|---|---|---|---|---|
| Calculés<br>Trouvés | C% | 63,0<br>62,8 | H% | 6,49<br>6,5 | Br% | 19,05<br>19,0 |

| Spectre IR ($CHCl_3$) | |
|---|---|
| Delta-4,6 3-one | 1654 $cm^{-1}$<br>1618 $cm^{-1}$<br>1584 $cm^{-1}$<br>878 $cm^{-1}$ |
| OH | 3611 $cm^{-1}$ |

**Préparation 2 : 17alpha-(3-bromo 1-propynyl) 11béta,17béta-dihydroxy 6-méthyl androsta-1,4,6-trièn-3-one.**

Stade A : 11béta,17béta-dihydroxy 17alpha-(3-tétrahydropyrannyloxy 1-propynyl) 3-éthyloxy 6-méthyl androsta-3,5-diène.

A 183 $cm^3$ d'une solution 1,6 M de méthyl lithium dans l'éther, refroidie à -5°/-7°C, on ajoute en 40 minutes une solution de 45,75 g de tétrahydro 2-(2-propynyloxy) 2H-pyranne dans 13 $cm^3$ d'éther éthylique. On agite 30 minutes à -5°/-7°C puis on ajoute en 50 minutes une solution de 22,5 g de 11béta-hydroxy 3-éthyloxy 6-méthyl androsta 3,5-dièn-17-one (préparé selon le brevet français 2.430.952) dans 157 $cm^3$ de tétrahydrofuranne, on agite 15 heures à température ambiante. On ajoute à 0°/+5°C une solution saturée de chlorure d'ammonium, décante et extrait avec de l'acétate d'éthyle. On concentre à sec sous pression réduite, on recueille 70 g de produit que l'on chromatographie sur silice en éluant successivement avec cyclohexane-acétate d'éthyle (8-2, 7-3, 6-4, 5-5) triéthylamine 1 pour mille), on obtient ainsi 22,6 g de produit utilisé tel quel pour le stade suivant.

Stade B : 11béta,17béta-dihydroxy 17alpha-(3-hydroxy 1-propynyl) 6-méthyl androsta-1,4,6-trièn-3-one.

a) Oxydation.

A un mélange de 37,7 g de dichlorodicyano hydroquinone et 867 $cm^3$ de toluène, on ajoute, en 5 minutes à 20/22°C, une solution de 22,6 g de produit obtenu au stade A dans 406 $cm^3$ de toluène. Après 1 heure d'agitation, on verse le mélange réactionnel sur 1 litre de solution aqueuse saturée de bicarbonate de sodium, sépare par décantation et lave à l'eau saturée de bicarbonate de sodium, puis avec une solution aqueuse à 15 pour cent de thiosulfate de sodium. On concentre la fraction organique à sec sous pression réduite et isole 19,2 g de produit intermédiaire 3-céto-1,4,6-triène.

b) Hydrolyse.

On agite pendant 2 heures, à température ambiante, le produit obtenu ci-dessus dans 288 $cm^3$ de méthanol et 96 $cm^3$ d'acide chlorhydrique 2N glacé, puis ajoute lentement 150 $cm^3$ d'une solution saturée de bicarbonate de sodium. On extrait à l'acétate d'éthyle, amène à sec et chromatographie le résidu (16,3 g) sur silice (éluant : chlorure de méthylène-méthanol (8-2)), on obtient 12,63 g de produit recherché utilisé tel quel pour le stade suivant. 1,740 g du produit obtenu ci-dessus sont cristallisés dans le chlorure de méthylène puis recristallisés dans l'acétate d'éthyle permettant d'obtenir 1,36 g de produit pur. F -~ 175°C. $[alpha]_D$ = -93°5 ± 3° (C = 0,5 % Ethanol)

| Analyse pour $C_{23}H_{28}O_4$ : | | | | |
|---|---|---|---|---|
| Calculés<br>Trouvés | C% | 74,97<br>75,2 | H% | 7,65<br>7,8 |

| Spectre IR ($CHCl_3$) | |
|---|---|
| Cétone conjuguée | 1647 cm$^{-1}$<br>1596 cm$^{-1}$<br>891 cm$^{-1}$ |
| OH libre | 3609 cm$^{-1}$ |

Stade C : 17alpha-(3-bromo 1-propynyl) 11béta,17béta-dihydroxy 6-méthyl androsta-1,4,6-trièn-3-one.

On opère comme au stade D de la préparation 1 à partir de 10 g de produit obtenu au stade B ci-dessus. On obtient 8,813 g de produit recherché. 1,83 g du produit ci-dessus sont chromatographiés à nouveau sur silice (éluant : acétate d'éthyle-cyclohexane (8-2)). On obtient 794 mg de produit. $[alpha]_D$ = -93°5 ± 3° (C = 0,5 % Ethanol)

| Analyse pour $C_{23}H_{27}O_3Br$ : | | | | | | |
|---|---|---|---|---|---|---|
| Calculés<br>Trouvés | C% | 63,26<br>63,4 | H% | 6,53<br>6,7 | Br% | 17,03<br>17,4 |

**Spectre IR**

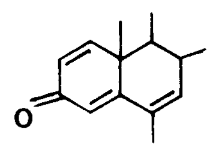

1651 cm$^{-1}$
1607 cm$^{-1}$ Max
1584 cm$^{-1}$ Ep.
891 cm$^{-1}$

OH 3611 cm$^{-1}$ (+ associé)

**Préparation 3 : N,N,N',N'-tétraéthyl 6-(1-pipérazinyl) 2,5-pyridinediamine.**

Stade A : 1-acétyl 4-[6-(diéthylamino) 3-nitro 2-pyridyl] pipérazine.

A un mélange de 78 g de 2,6-dichloro 3-nitro pyridine, 600 cm$^3$ d'acétonitrile et 66,3 g de carbonate de potassium, on ajoute en 50 minutes à 0°C, une solution de 51,22 g de N-acétyl pipérazine dans 200 cm$^3$ d'acétonitrile. On laisse revenir à température ambiante et agite 1 heure 15. On filtre les sels minéraux, ajoute au filtrat, 180 cm$^3$ de N-diéthylamine et 76 g de carbonate de potassium et chauffe le mélange 1 heure 15 au reflux, après refroidissement, on filtre les sels minéraux, et évapore à sec sous pression réduite, le résidu (166,9 g) est recristallisé dans 200 cm$^3$ d'acétate d'éthyle, on obtient 87,4 g de produit attendu. F = 120°C.

**Spectre IR** ($CHCl_3$)

>=O 1637 cm$^{-1}$
Système conjugué 1593 cm$^{-1}$
1ère bande $NO_2$ 1569 cm$^{-1}$ - 1510 cm$^{-1}$
2ème bande $NO_2$ 1346 ou 1299

Stade B : N,N,N',N'-tétraéthyl 6-(1-pipérazinyl) 2,5-pyridinediamine.

On hydrogène sous une pression maximum de 1250 mbar de mercure à 25 °C, un mélange de 70 g du produit obtenu au stade A, 1500 $cm^3$ de méthanol, 61,5 $cm^3$ d'acétaldéhyde et 10 g de charbon actif à 10 % de palladium. On absorbe environ 20 litres d'hydrogène, on filtre le catalyseur et évapore le filtrat à sec sous pression réduite. On reprend le résidu (89,8 g) avec 500 $cm^3$ de n-propanol et 91,3 g de potasse en pastilles, et chauffe 3 heures au reflux. On coule la solution refroidie dans 1 litre d'eau glacée et extrait avec du chlorure de méthylène, lave la solution organique avec une solution saturée de chlorure de sodium, sèche, filtre et concentre à sec, sous pression réduite. On chromatographie le résidu (58,9 g) sur silice (éluant : chlorure de méthylène-méthanol-ammoniaque (95-5-0,5)). On obtient 48,35 g de produit attendu que l'on utilise tel quel.

| Analyse pour $C_{17}H_{31}N_5$ | | | | | | |
|---|---|---|---|---|---|---|
| Calculés<br>Trouvés | C% | 66,84<br>66,9 | H% | 10,23<br>10,5 | N% | 22,93<br>22,6 |

Spectre IR ($CHCl_3$)

C = C ⎫ 1596 $cm^{-1}$
C = N ⎬ 1560 $cm^{-1}$
Hétéroaromatique ⎪ 1531 $cm^{-1}$
⎭ 1487 $cm^{-1}$

**EXEMPLE 1 : Méthane sulfonate de 17alpha-[3-[4-[(2,6-bis(1-pyrrolidinyl) 4-pyrimidinyl] 1-pipérazinyl] 1-propynyl] 11béta,17béta-dihydroxy androsta-4,6-dièn-3-one.**

A une solution de 4,741 g de produit obtenu au stade D de la préparation 1, on ajoute 6,834 g de 2,4-bis(1-pyrrolidinyl) 6-(1-pipérazinyl) pyrimidine (préparé selon brevet WO/87-01706) et 3,180 g de carbonate de potassium. On agite pendant 1 heure, puis filtre l'insoluble et concentre à sec sous pression réduite. On chromatographie le résidu sur silice (éluant : chlorure de méthylène-méthanol-ammoniaque (95-5-0,5)). On obtient 6,52 g de produit que l'on dissout dans 500 $cm^3$ de chlorure de méthylène, filtre l'insoluble, ajoute 100 $cm^3$ d'isopropanol et évapore le chlorure de méthylène. On glace à 0 °C la suspension, essore et obtient 5,66 g de base libre. F = 266 °C.

Salification :

On dissout 1 g de la base obtenue ci-dessus dans 600 $cm^3$ de méthanol et ajoute 10,4 $cm^3$ d'une solution 0,3 M d'acide méthane sulfonique dans l'acétate d'éthyle. On concentre sous pression réduite jusqu'à 100 $cm^3$ en remplaçant le méthanol par ajout d'acétate d'éthyle pendant la distillation. On essore le sel cristallisé de l'acétate d'éthyle à 0 °C et on obtient 1,163 g de produit recherché. F = 225 °C (peu net).

| Analyse pour $C_{38}H_{52}O_3N_6$, $2CH_3SO_3H$ : | | | | | | | | |
|---|---|---|---|---|---|---|---|---|
| Calculés<br>Trouvés | C% | 57,66<br>57,35 | H% | 7,25<br>7,3 | N% | 10,08<br>9,9 | S% | 7,69<br>7,7 |

| Spectre IR ($CHCl_3$) | |
|---|---|
| OH | 3610 $cm^{-1}$ (+ associé) |
| Delta-4,6 3-one | 1654 $cm^{-1}$<br>1617 $cm^{-1}$<br>878 $cm^{-1}$ |
| Système conjugué de la copule | 1559 $cm^{-1}$ (F)<br>1552 $cm^{-1}$ (F)<br>1487 $cm^{-1}$ (F) |

**EXEMPLE 2 : 11béta,17béta-dihydroxy 17alpha-[3-[4-[(2,6-bis(1-pyrrolidinyl) 4-pyrimidinyl] 1-pipérazinyl] 1-propynyl] 6-méthyl androsta-1,4,6-trièn-3-one.**

On agite pendant 3 heures 45 minutes à température ambiante un mélange de 1,509 g du produit obtenu au stade C de la préparation 2 dans 22 $cm^3$ d'acétone, 2,116 g de 2,4-bis(1-pyrrolidinyl) 6-(1-pipérazinyl) pyrimidine (préparé selon le brevet WO 87/01706) et 0,986 g de carbonate de potassium. On filtre l'insoluble, le lave à l'acétone puis avec du chlorure de méthylène à 5 % de méthanol. On amène à sec le filtrat, chromatographie le résidu sur silice (éluant : chlorure de méthylène-méthanol (95-5)) et obtient 1,872 g de produit attendu brut. Au produit obtenu ci-dessus, on ajoute 741 mg d'une préparation précédente et chromatographie le tout sur silice (éluant : acétate d'éthyle-méthanol (95-5)). L'extrait sec recueilli (2,331 g) est recristallisé dans 10 $cm^3$ d'acétate d'éthyle. On obtient 2,2 g de produit recherché. F -~ 275 ° C.

| Analyse pour $C_{39}H_{52}O_3N_6$ : | | | | | | |
|---|---|---|---|---|---|---|
| Calculés<br>Trouvés | C% | 71,74<br>71,7 | H% | 8,02<br>8,2 | N% | 12,87<br>12,5 |

Spectre IR ($CHCl_3$)

OH 3609 $cm^{-1}$

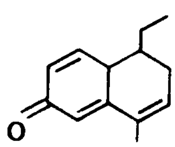

1651 $cm^{-1}$

1607 $cm^{-1}$

891 $cm^{-1}$

Système conjugué 1564 $cm^{-1}$ (Max. F.)

1554 $cm^{-1}$ (ep.)

1522 $cm^{-1}$ (ep.)

1487 $cm^{-1}$ (ep.)

**EXEMPLE 3 : 17alpha-[3-[4-[5,6-bis(diéthylamino) 2-pyridyl] 1-pipérazinyl] 1-propynyl] 11béta,17béta-dihydroxy 6-méthyl androsta-1,4,6-trièn-3-one.**

On opère comme à l'exemple 2 à partir de 2 g du produit du stade C de la préparation 2 et en utilisant 2,828 g de N,N,N',N'-tétraéthyl 6-(1-pipérazinyl) 2,3-pyridinediamine (préparée selon le brevet WO 87/01706). On obtient 4,684 g de produit brut que l'on chromatographie sur silice (éluant : chlorure de méthylène-méthanol (95-5)) puis acétate d'éthyle-méthanol (95-5)). On obtient 2,65 g de produit recherché. $[alpha]_D$ = -72 ° 5 ± 2 ° 5 (C = 0,5 % Ethanol)

| Analyse pour $C_{40}H_{57}O_3N_5$ : | | | | | | |
|---|---|---|---|---|---|---|
| Calculés<br>Trouvés | C% | 72,43<br>72,3 | H% | 8,77<br>8,8 | N% | 10,22<br>10,1 |

<u>Spectre IR</u> ($CHCl_3$)

OH 3611 $cm^{-1}$

Cétone conjuguée 1651 $cm^{-1}$

1607 $cm^{-1}$ Type 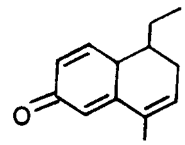

891 $cm^{-1}$

Système conjugué 1586 $cm^{-1}$

1565 $cm^{-1}$

1477 $cm^{-1}$

**<u>EXEMPLE 4</u> : 17alpha-[3-[4-[3,6-bis(diéthylamino) 2-pyridyl] 1-pipérazinyl] 1-propynyl] 11béta,17béta-dihydroxy 6-méthyl androsta-1,4,6-trièn-3-one.**

On opère comme à l'exemple 3, à partir de 2 g de produit obtenu au stade C de la préparation 2 et en utilisant 2,83 g de N,N,N',N'-tétraéthyl 6-(1-pipérazinyl) 2,5-pyridinediamine (préparée selon la demande de brevet n° 89-03740 et obtenue selon le stade B de la préparation 3). On obtient 1,75 g de produit attendu que l'on chromatographie une 3ème fois sur silice (éluant : acétate d'éthyle-méthanol (96-4)). On isole 1,458 g de produit recherché.

$[alpha]_D$ = -83°5 ± 2°5 (C = 0,5 % Ethanol)

| Analyse pour $C_{40}H_{57}O_3N_5$ : | | | | | | |
|---|---|---|---|---|---|---|
| Calculés<br>Trouvés | C% | 72,8<br>72,6 | H% | 8,76<br>8,8 | N% | 10,43<br>10,4 |

<u>Spectre IR</u> ($CHCl_3$)

OH 3611 $cm^{-1}$

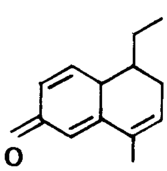

1651 $cm^{-1}$

1606 $cm^{-1}$

891 $cm^{-1}$

Système conjugué 1595 $cm^{-1}$

1561 $cm^{-1}$

1486 $cm^{-1}$

<u>ETUDE PHARMACOLOGIQUE</u> : activité antioxydante.

L'activité antioxydante est recherchée, in vitro, par le test de formation de la malonyldialdéhyde (MDA) qui mesure la péroxydation lipidique déclenchée

**soit a)** de fa on non enzymatique par le sulfate ferreux, dans

1) des homogénats de cerveau ou
2) des microsomes hépatiques de rat

**soit b)** de façon enzymatique par NADPH et le tétrachlorure de carbone, dans des microsomes hépatiques de rat.

**1.1 :** La formation de MDA est mesurée sur des homogénats au 1/10$^e$ (V/V) de cerveaux de rat S.D (200 g) préparés dans un tampon de Krebs pH 7.4 selon les conditions décrites dans J. Biol. Chem. 262 (1987) 10438-10440. On incube 1 ml d'homogénat pendant 60 mn à 37 ° C en présence de 25 microlitres d'éthanol ou d'eau ou d'un mélange des deux, selon le produit, contenant ou non le produit à tester, après addition de 25 microlitres de solution de sulfate ferreux préparée extemporanément dans de l'eau dégazée à l'argon (200 micromoles final). On prélève 0,25 ml de mélange incubé et ajoute 1,5 ml d'acide phosphorique à 1 %, 0,25 ml de solution 200 micromoles de déféroxamine (Desféral $^R$ Ciba Geigy) dans l'eau, 10 microlitres de tert-butylhydroxytoluène (BHT) à 8,7 mg/ml dans l'éthanol et 0,5 ml d'acide thiobarbiturique (TBA) à 0,6 % dans l'eau. On chauffe le mélange à 100 ° C pendant 45 mn, refroidit, ajoute 4 ml de n-butanol, centrifuge pendant 15 mn à 4000 t/mn puis lit la DO à 535 nm de la fraction surnageante. On incube dans les mêmes conditions les blancs de réaction en absence de $Fe^{++}$. On calcule le pourcentage d'inhibition :

$$\text{pourcentage d'inhibition} = \frac{\text{DO en présence de produit}}{\text{DO en absence de produit}}$$

Résultats :

| concentration | pourcentage d'inhibition | | |
|---|---|---|---|
| produit testé | $5.10^{-4}$ M | $1.10^{-4}$ M | $1.10^{-5}$ M |
| produit obtenu à l'exemple 1 | 60,8 ± 3,0 | 9,8 ± 11,2 | -10,6 ± 9,0 |
| produit obtenu à l'exemple 2 | 53,1 ± 7,8 | 26,6 ± 3,9 | 28,0 ± 4,1 |
| produit obtenu à l'exemple 3 | 64,2 ± 10 | 62,9 ± 4,5 | 51,3 ± 13,7 |
| produit obtenu à l'exemple 4 | 65,4 ± 10,1 | 51,0 ± 12,4 | 48,5 ± 13,1 |

**1.2 :** La formation de MDA est mesurée sur des microsomes hépatiques de rat S.D (200 g) préparés à partir de la fraction sédimentée à 100.000 G d'un homogénat de foie dans un tampon sucrose dont on utilise la fraction restant insoluble à 100.000 G dans un tampon de pyrophosphate de sodium 100 mM pH 7.4 et que l'on homogénéise dans un tampon phosphate de sodium 100 mM pH 7.4 à 20 % de glycérol, puis que l'on conserve à -80 ° C.

On incube les microsomes pendant 15 mn à 37 ° C dans 1 ml contenant du tampon Tris, HCl 35 mM, KCl 0,1 M pH 7.4, 1 mg de protéine microsomale, 5 microlitres d'éthanol renfermant ou non le produit à tester, 250 microlitres de solution d'ascorbate dans le tampon Tris (0.5 mM final), après l'addition de 250 microlitres de sulfate ferreux préparée extemporanément dans le tampon Tris (6 micromoles final). On arrête la réaction par addition de 2 ml d'une solution d'acide trichloracétique 1 M dans l'acide chlorhydrique 0,25 M contenant 0.4 % d'acide thiobarbiturique. On chauffe le mélange à 85 ° C pendant 25 mn, refroidit, centrifuge pendant 15 mn à 3500 t/mn puis lit la DO à 535 nm de la fraction surnageante. On effectue en même temps les blancs de réaction en absence de $Fe^{++}$. On calcule le pourcentage d'inhibition comme précédemment.

Résultats :

| concentration | pourcentage d'inhibition | | | |
|---|---|---|---|---|
| produit testé | $5.10^{-5}$ M | $1.10^{-5}$ M | $5.10^{-6}$ M | $1.10^{-6}$ M |
| produit de l'exemple 1 | 97,5 ± 0,5 | 46 ± 3 | 19,5 ± 6 | 0 ± 18 |
| produit de l'exemple 2 | 85 ± 1,5 | 11,3 ± 2 | 0 ± 3 | 0 ± 2 |
| produit de l'exemple 3 | | 95,5 | 99,5 | 55 ± 9 |
| produit de l'exemple 4 | | 100 | 99,5 | 51 ± 3 |

**2 :** La formation de MDA est mesurée sur des microsomes hépatiques de rats prétraités au phénobarbital (80 mg/kg par injection intrapéritonéale quotidienne pendant 4 jours), préparés comme décrit précédemment. On incube les microsomes pendant 15 mn à 37 °C dans 1 ml contenant du tampon phosphate 0,1 M pH 7.4, 1 mg de protéine microsomale, 5 microlitres d'éthanol renfermant ou non le produit à tester, 5 microlitres de tétrachlorure de carbone (5,5 mM final) après l'addition de 50 microlitres de solution de NADPH dans le tampon phosphate (1 mM final). On arrête la réaction puis effectue le dosage selon les conditions décrites précédemment.

Résultats :

| concentration | pourcentage d'inhibition | | |
|---|---|---|---|
| produit testé | $1.10^{-5}$ M | $5.10^{-6}$ M | $1.10^{-6}$ M |
| produit de l'exemple 1 | 86 ± 2 | 75 ± 4 | 16 ± 1 |
| produit de l'exemple 2 | 80 ± 1 | 61 ± 1,5 | 22 ± 15 |
| produit de l'exemple 3 | 93 | 89 ± 2 | 48 ± 2 |
| produit de l'exemple 4 | 91 ± 2 | 85 | 49 ± 2 |

**Revendications**

**1.** Les composés de formule (I) :

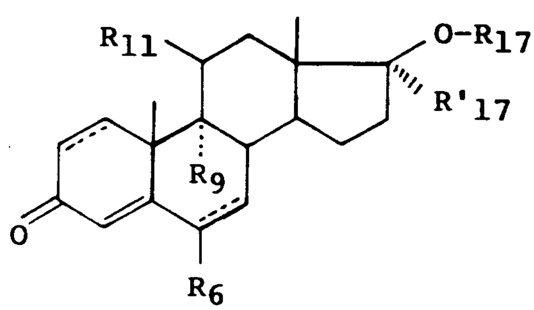

(I)

dans laquelle :

- $R_6$ représente un atome d'hydrogène, un radical méthyle, un atome de fluor ou un atome de chlore,
- $R_9$ et $R_{11}$ forment ensemble une seconde liaison entre les carbones $C_9$ et $C_{11}$ ou $R_9$ est un atome d'hydrogène ou un atome de fluor et $R_{11}$ est un atome d'hydrogène, un radical hydroxyle ou un radical oxo,
- les traits pointillés, dans les cycles A et B, indiquent la présence éventuelle d'une liaison supplémentaire en position 1(2) et 6(7)

et dans laquelle $R_{17}$ est un atome d'hydrogène ou un radical acyle dérivé d'un acide organique carboxylique renfermant de 1 à 18 atomes de carbone et $R'_{17}$ est un radical

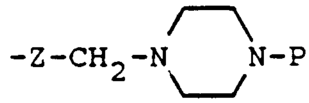

dans lequel Z est une simple liaison, un radical alcénylène ou un radical alcynylène renfermant de 2 à 5 atomes de carbone et P représente un radical pyrimidinyle ou un radical pyridyle, éventuellement substitué par un ou deux radicaux identiques ou différents choisis parmi les radicaux amino, alkyl ou dialkylamino ou les hétérocycles aminés à 5 ou 6 chaînons, substitués par un radical alkyle ayant de 1 à 3 atomes de carbone, et les sels de ces composés.

**2.** Les composés de formule (I) telle que définie à la revendication 1 pour lesquels P représente un radical pyrimidinyle ou un radical pyridyle substitué par deux radicaux identiques ou différents choisis parmi les radicaux dialkylamino ou les hétérocycles aminés à 5 ou 6 chaînons.

**3.** Les composés de formule (I) telle que définie à la revendication 1 ou 2 pour lesquels P représente un radical 2,6-bis(1-pyrrolidinyl) 4-pyrimidinyle, un radical 5,6-bis(diéthylamino) 2-pyridyle ou un radical 3,6-bis(diéthylamino) 2-pyridyle.

**4.** Les composés de formule (I) telle que définie à l'une quelconque des revendications 1 à 3 pour lesquels $R_6$ est un atome d'hydrogène.

**5.** Les composés de formule (I) telle que définie à l'une quelconque des revendications 1 à 3 pour lesquels $R_6$ est un radical méthyle.

**6.** Les composés de formule (I) telle que définie à l'une quelconque des revendications 1 à 5 pour lesquels le trait en pointillé en position 6(7) indique la présence d'une seconde liaison.

**7.** Les composés de formule (I) telle que définie à l'une quelconque des revendications 1 à 6 pour lesquels $R_9$ est un atome d'hydrogène et $R_{11}$ est un radical hydroxyle.

**8.** Les composés de formule (I) telle que définie à l'une quelconque des revendications 1 à 7 pour lesquels le trait en pointillé en position 1(2) indique la présence d'une seconde liaison.

**9.** Les composés de formule (I) telle que définie à l'une des revendications 1 à 8 pour lesquels $R_{17}$ est un atome d'hydrogène.

**10.** Les composés de formule (I) telle que définie à l'une quelconque des revendications 1 à 9 pour lesquels $R'_{17}$ représente un radical

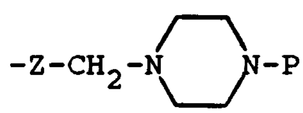

dans lequel Z représente un radical -C≡C-.

**11.** Les composés de formule (I) telle que définie à l'une quelconque des revendications 1 à 10 et répondant à l'une quelconque des formules :

- 17alpha-[3-[4-[2,6-bis(1-pyrrolidinyl) 4-pyrimidinyl] 1-pipérazinyl] 1-propynyl] 11béta,17béta-dihydroxy-androsta 4,6-dièn-3-one, ou
- 17alpha-[3-[4-[2,6-bis(1-pyrrolidinyl) 4-pyrimidinyl] 1-pipérazinyl] 1-propynyl] 11béta,17béta-dihydroxy 6-méthyl-androsta-1,4,6-trièn-3-one ou
- 17alpha-[3-[4-[5,6-bis(diéthylamino) 2-pyridyl] 1-pipérazinyl] 1-propynyl] 11béta,17béta-dihydroxy 6-méthyl androsta-1,4,6-trièn-3-one ou
- 17alpha-[3-[4-[3,6-bis(diéthylamino) 2-pyridyl] 1-pipérazinyl] 1-propynyl] 11béta,17béta-dihydroxy 6-méthyl androsta-1,4,6-trièn-3-one et leurs sels.

**12.** Procédé de préparation des composés de formule (I) telle que définie à la revendication 1, caractérisé en ce que l'on soumet, dans un solvant neutre et en présence d'une base, un composé de formule (II) :

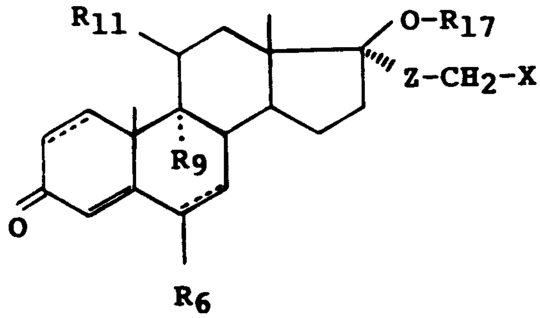

(II)

dans laquelle X est un atome d'halogène et $R_6$, $R_9$, $R_{11}$, les traits pointillés, $R_{17}$ et Z ont la signification indiquée à la revendication 1, à l'action d'un composé de formule (III) :

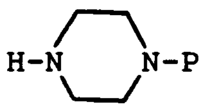

(III)

dans laquelle P a la même signification qu'à la revendication 1 et, si désiré, l'on soumet les produits de formule (I) à l'action d'un acide pour obtenir le sel correspondant.

**13.** A titre de médicament, les composés de formule (I) telle que définie à l'une quelconque des revendications 1 à 10 et leurs sels avec les acides pharmaceutiquement acceptables.

**14.** A titre de médicament, selon la revendication 13, l'un quelconque des composés définis à la revendication 11 et leurs sels avec les acides pharmaceutiquement acceptables.

**15.** Les compositions pharmaceutiques renfermant comme principe actif au moins l'un quelconque des médicaments tel que défini à la revendication 13 ou 14.

**16.** A titre de produits industriels nouveaux, les composés de formule (II).

**17.** Les produits industriels nouveaux, selon la revendication 16, répondant à la formule :
- 17alpha-(3-bromo-1-propynyl) 11béta,17béta-dihydroxy-androsta-4,6-dièn-3-one
  ou à la formule :
- 17alpha-(3-bromo-1-propynyl) 11béta,17béta-dihydroxy 6-méthylandrosta-1,4,6-trièn-3-one.

**Claims**

**1.** The compounds of formula (I):

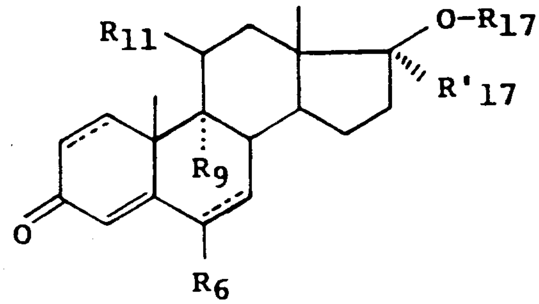

(I)

in which:

- $R_6$ represents a hydrogen atom, a methyl radical, a fluorine atom or a chlorine atom,
- $R_9$ and $R_{11}$ together form a second bond between the carbons $C_9$ and $C_{11}$ or $R_9$ is a hydrogen atom or a fluorine atom and $R_{11}$ is a hydrogen atom, a hydroxyl radical or an oxo radical,
- the dotted lines, in rings A and B, indicate the optional presence of an additional bond in position 1(2) and 6(7),

and in which $R_{17}$ is a hydrogen atom or an acyl radical derived from an organic carboxylic acid containing 1 to 18 carbon atoms and $R'_{17}$ is a radical

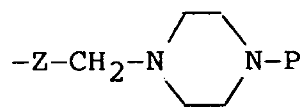

in which Z is a single bond, an alkenylene radical or an alkynylene radical containing 2 to 5 carbon atoms and P represents a pyrimidinyl radical or a pyridyl radical, optionally substituted by one or two identical or different radicals chosen from amino, alkyl or dialkylamino radicals or amino heterocycles with 5 or 6 members, substituted by an alkyl radical having 1 to 3 carbon atoms, and the salts of these compounds.

**2.** The compounds of formula (I) as defined in claim 1 for which P represents a pyrimidinyl radical or a pyridyl radical substituted by two identical or different radicals chosen from dialkylamino radicals or amino heterocycles with 5 or 6 members.

**3.** The compounds of formula (I) as defined in claim 1 or 2 for which P represents a 2,6-bis(1-pyrrolidinyl) 4-pyrimidinyl radical, a 5,6-bis(diethylamino) 2-pyridyl radical or a 3,6-bis(diethylamio) 2-pyridyl radical.

**4.** The compounds of formula (I) as defined in any one of claims 1 to 3 for which $R_6$ is a hydrogen atom.

**5.** The compounds of formula (I) as defined in any one of claims 1 to 3 for which $R_6$ is a methyl radical.

**6.** The compounds of formula (I) as defined in any one of claims 1 to 5 for which the dotted line in position 6(7) indicates the presence of a second bond.

**7.** The compounds of formula (I) as defined in any one of claims 1 to 6 for which $R_9$ is a hydrogen atom and $R_{11}$ is a hydroxyl radical.

**8.** The compounds of formula (I) as defined in any one of claims 1 to 7 for which the dotted line in position 1(2) indicates the presence of a second bond.

**9.** The compounds of formula (I) as defined in one of claims 1 to 8 for which $R_{17}$ is a hydrogen atom.

**10.** The compounds of formula (I) as defined in any one of claims 1 to 9 for which $R'_{17}$ represents a radical

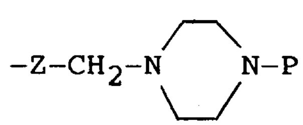

in which Z represents a -C≡C- radical.

**11.** The compounds of formula (I) as defined in any one of claims 1 to 10 and corresponding to any one of the formulae:

- 17alpha-[3-[4-[2,6-bis(1-pyrrolidinyl) 4-pyrimidinyl] 1-piperazinyll 1-propynyl] 11beta,17beta-dihydroxy-androsta 4,6-dien-3-one, or

- 17alpha-[3-[4-[2,6-bis(1-pyrrolidinyl) 4-pyrimidinyl] 1-piperazinyl] 1-propynyl] 11beta,17beta-dihydroxy 6-methyl androsta-1,4,6-trien-3-one or
- 17alpha-[3-[4-[5,6-bis(diethylamino) 2-pyridyl] 1-piperazinyl] 1-propynyl] 11beta,17beta-dihydroxy 6-methyl androsta-1,4,6-trien-3-one or
- 17alpha-[3-[4-[3,6-bis(diethylamino) 2-pyridyl] 1-piperazinyl] 1-propynyl] 11beta,17beta-dihydroxy 6-methyl androsta-1,4,6-trien-3-one

and their salts.

**12.** Preparation process for the compounds of formula (I) as defined in claim 1, characterized in that a compound of formula (II):

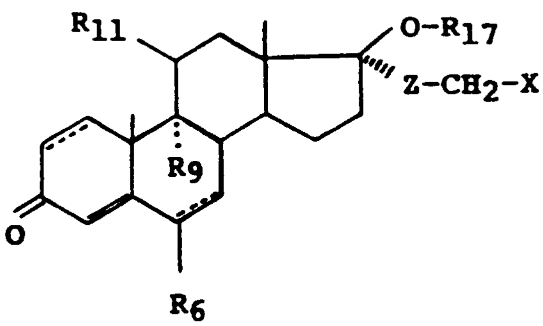

(II)

in which X is a halogen atom and $R_6$, $R_9$, $R_{11}$, the dotted lines, $R_{17}$ and Z have the meaning indicated in claim 1, is subjected, in a neutral solvent and in the presence of a base, to the action of a compound of formula (III):

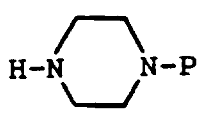

(III)

in which P has the same meaning as in claim 1 and, if desired, the products of formula (I) are subjected to the action of an acid in order to obtain the corresponding salt.

**13.** As medicaments, the compounds of formula (I) as defined in any one of claims 1 to 10 and their salts with pharmaceutically acceptable acids.

**14.** As a medicament, according to claim 13, any one of the compounds defined in claim 11 and their salts with pharmaceutically acceptable acids.

**15.** The pharmaceutical compositions containing as active ingredient at least any one of the medicaments as defined in claim 13 or 14.

**16.** As new industrial products, the compounds of formula (II).

**17.** The new industrial products, according to claim 16, corresponding to formula:

- 17alpha-(3-bromo-1-propynyl) 11beta,17beta-dihydroxy-androsta-4,6-dien-3-one

or to formula:

- 17alpha-(3-bromo-1-propynyl) 11beta,17beta-dihydroxy 6-methyl androsta-1,4,6-trien-3-one.

**Patentansprüche**

1. Verbindungen der Formel (I)

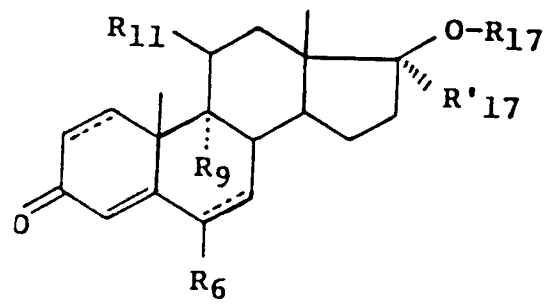

(I)

worin:
- $R_6$ ein Wasserstoffatom, einen Methylrest, ein Fluoratom oder ein Chloratom darstellt,
- $R_9$ und $R_{11}$ bilden zusammen eine zweite Bindung zwischen den $C_9$- und $C_{11}$-Kohlenstoffen oder $R_9$ ist ein Wasserstoffatom oder ein Fluoratom und $R_{11}$ ist ein Wasserstoffatom, ein Hydroxylrest oder ein Oxorest,
- die punktierten Linien in den Ringen A und B zeigen die eventuelle Anwesenheit einer zusätzlichen Bindung in 1(2)- und 6(7)-Stellung an,

und worin $R_{17}$ ein Wasserstoffatom oder ein Acylrest, abgeleitet von einer organischen Carbonsäure mit 1 bis 18 Kohlenstoffatomen, ist, und $R'_{17}$ ist ein Rest

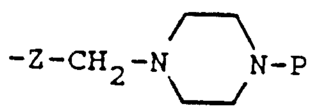

worin Z eine Einfachbindung ist, ein Alhenylenrest oder ein Alkinylenrest mit 2 bis 5 Kohlenstoffatomen und P bedeutet einen Pyrimidinylrest oder einen Pyridylrest, gegebenenfalls substituiert durch ein oder zwei identische oder verschiedene Reste, ausgewählt unter den Resten Amino, Alkyl oder Dialkylamino oder den Aminheterocyclen mit 5 oder 6 Kettengliedern, substituiert durch einen Alkylrest mit 1 bis 3 Kohlenstoffatomen, und die Salze dieser Verbindungen.

2. Verbindungen der Formel (I), wie in Anspruch 1 definiert, für die P einen Pyrimidinylrest oder einen Pyridylrest, substituiert durch zwei identische oder verschiedene Reste, ausgewählt unter den Dialkylaminoresten oder den Aminheterocyclen mit 5 oder 6 Kettengliedern, bedeutet.

3. Verbindungen der Formel (I), wie in Anspruch 1 oder 2 definiert, für die P einen 2,6-Bis-(1-pyrrolidinyl)-4-pyrimidinyl-, einen 5,6-Bis-(diethylamino)-2-pyridyl- oder einen 3,6-Bis-(diethylamino)-2-pyridylrest bedeutet.

4. Verbindungen der Formel (I), wie in einem der Ansprüche 1 bis 3 definiert, worin $R_6$ ein Wasserstoffatom ist.

5. Verbindungen der Formel (I), wie in einem der Ansprüche 1 bis 3 definiert, worin $R_6$ ein Methylrest ist.

6. Verbindungen der Formel (I), wie in einem der Ansprüche 1 bis 5 definiert, worin die punktierte Linie in 6(7)-Stellung die Anwesenheit einer zweiten Bindung anzeigt.

7. Verbindungen der Formel (I), wie in einem der Ansprüche 1 bis 6 definiert, worin $R_9$ ein Wasserstoffatom und $R_{11}$ ein Hydroxylrest ist.

8. Verbindungen der Formel (I), wie in einem der Ansprüche 1 bis 7 definiert, worin die punktierte Linie in 1(2)-Stellung die Anwesenheit einer zweiten Bindung anzeigt.

9. Verbindungen der Formel (I), Wie in einem der Ansprüche 1 bis 8 definiert, worin $R_{17}$ ein Wasserstoffatom ist.

10. Verbindungen der Formel (I), wie in einem der Ansprüche 1 bis 9 definiert, worin $R'_{17}$ einen Rest

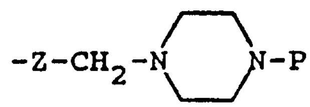

bedeutet, worin Z einen Rest -C≡C-darstellt.

11. Verbindungen der Formel (I), wie in einem der Ansprüche 1 bis 10 definiert, entsprechend einer der Formeln:
   - 17α-[3-[4-[2,6-Bis-(1-pyrrolidinyl)-4-pyrimidinyl]-1-piperazinyl]-1-propinyl]-11β,17β-dihydroxy-androsta-4,6-dien-3-on oder
   - 17α-[3-[4-[2,6-Bis-(1-pyrrolidinyl)-4-pyrimidinyl]-1-piperazinyl]-1-propinyl]-11β,17β-dihydroxy-6-methyl-androsta-1,4,6-trien-3-on oder
   - 17α-[3-[4-[5,6-Bis-(diethylamino)-2-pyridyl]-1-piperazinyl]-1-propinyl]-11β,17β-dihydroxy-6-methylandrosta-1,4,6-trien-3-on oder
   - 17α-[3-[4-[3,6-Bis-(diethylamino)-2-pyridyl]-1-piperazinyl]-1-propinyl]-11β,17β-dihydroxy-6-methyl-androsta-1,4,6-trien-3-on

   und ihre Salze.

12. Verfahren zur Herstellung der Verbindungen der Formel (I), wie in Anspruch 1 definiert, dadurch gekennzeichnet, daß man in einem neutralen Lösungsmittel und in Anwesenheit einer Base eine Verbindung der Formel (II)

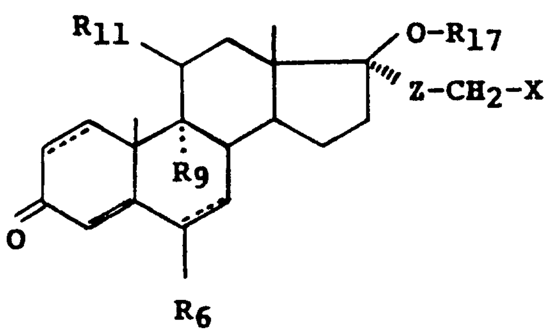

(II)

worin X ein Halogenatom ist und $R_6$, $R_9$, $R_{11}$, die punktierten Linien, $R_{11}$ und Z die in Anspruch 1 angegebene Bedeutung haben, der Einwirkung einer Verbindung der Formel (III)

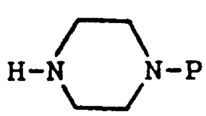

(III)

unterwirft, worin P die gleiche Bedeutung wie in Anspruch 1 hat, und daß man gewünschtenfalls die Produkte der Formel (I) der Einwirkung einer Säure unterwirft, um das entsprechende Salz zu erhalten.

13. Als Arzneimittel die Verbindungen der Formel (I), wie in einem der Ansprüche 1 bis 10 definiert, und ihre Salze mit pharmazeutisch annehmbaren Säuren.

**14.** Als Arzneimittel gemäß Anspruch 13 eine der in Anspruch 11 definierten Verbindungen und ihre Salze mit pharmazeutisch annehmbaren Säuren.

**15.** Pharmazeutische Zusammensetzungen, enthaltend als Wirkstoff mindestens ein Arzneimittel, wie in Anspruch 13 oder 14 definiert.

**16.** Als neue industrielle Produkte die Verbindungen der Formel (II).

**17.** Neue industrielle Produkte gemäß Anspruch 16, entsprechend der Formel:
- 17α-[3-Brom-1-propinyl)-11β,17β-dihydroxy-androsta-4,6-dien-3-on, oder entsprechend der Formel:
- 17α-[3-Brom-1-propinyl)-11β,17β-dihydroxy-6-methyl-androsta-1,4,6-trien-3-on.